# EUROPÄISCHE PATENTSCHRIFT

(11) **EP 1 868 518 B1**
(45) Veröffentlichungstag und Bekanntmachung des Hinweises auf die Patenterteilung: **17.11.2010**
(21) Anmeldenummer: 06754740.6
(22) Anmeldetag: 13.04.2006
(51) Int. Cl.: A61B 17/86

(54) **KNOCHENSCHRAUBE, INSBESONDERE ANKERSCHRAUBE FÜR SPONGIÖSES KNOCHENGEWEBE**
BONE SCREW, ESPECIALLY ANCHOR SCREW FOR SPONGY BONE TISSUE
VIS A OS, EN PARTICULIER VIS D'ANCRAGE POUR TISSUS OSSEUX SPONGIEUX

(30) Priorität: 15.04.2005 DE 202005006076 U
(43) Veröffentlichungstag der Anmeldung: 26.12.2007
(73) Patentinhaber: Königsee Implantate und Instrumente zur Ostheosynthese GmbH, 07426 Königsee-Aschau (DE)
(72) Erfinder: GEYER, Michael, 87459 Pfronten (DE)
(74) Vertreter: Meissner, Bolte & Partner
(86) Internationale Anmeldenummer: PCT/EP2006/061605
(87) Internationale Veröffentlichungsnummer: WO 2006/108875

(56) Entgegenhaltungen:
- EP-A- 1 584 296
- FR-A- 2 732 211
- US-A- 6 030 162
- US-A- 6 096 060
- US-A1- 2003 045 881
- US-B2- 6 511 499

## Beschreibung

Die Erfindung betrifft eine Ankerschraube für spongiöses Knochengewebe zur Verankerung für Nahtfäden und für eine Tenodese nach dem Oberbegriff des Anspruchs 1.

Knochenschrauben, insbesondere Ankerschrauben zum Befestigen von Nähten bzw. Nahtfäden verschiedener Art und/oder für eine Tenodese sind bekannt. In der medizinischen Praxis müssen derartige Knochenschrauben sehr häufig in spongiöses Knochengewebe eingebracht werden. Ein derartiges Knochengewebe ist durch eine schwammartige Gerüststruktur gekennzeichnet, wobei in die dazwischen liegenden Hohlräume Knochenmark eingelagert ist. Ein Beispiel hierfür ist die Trabekelstruktur des Oberschenkelknochens im Bereich des Oberschenkelhalses. Derartige Knochenschrauben müssen im spongiösen Knochengewebe fest verankert sein und Aufnahme- bzw. Befestigungsmöglichkeiten aufweisen.

Bekannte sogenannte Fadenanker besitzen eine Befestigungsöse, die einen OP-Faden aufnehmen kann. Ein Beispiel ist aus US-B-6511499 bekannt. Die bekannten Fadenanker werden unter die Kortikalis versenkt und sind im Regelfall nicht zu einem späteren Zeitpunkt entfernbar. Dies stellt sich als wesentlicher Nachteil dar. Aus FR-A-2732211 ist eine Ankerschraube bekannt, die die Merkmalskombination des Oberbegriffs des Anspruchs 1 aufweist. Es ist die Aufgabe der Erfindung, eine weiterentwickelte Ankerschraube für spongiöses Knochengewebe anzugeben, welche eine Vielzahl von Fäden aufnehmen kann, so dass die Zahl notwendiger Anker reduziert ist.

Diese Aufgabe wird mit einer Knochenschraube nach den Merkmalen des Anspruchs 1 gelöst, wobei die Unteransprüche zweckmäßige bzw. vorteilhafte Ausgestaltungen enthalten.

Erfindungsgemäß ist die Knochenschraube durch einen Schraubenkörper gekennzeichnet, der einen Gewindedurchmesser im Bereich von 3 bis 15 Millimeter und eine dem zwei- bis zehnfachen des Gewindedurchmessers entsprechende Länge in Verbindung mit mindestens einem auf der Längsachse des Schraubenkörpers angeordneten gewindefreien Abschnitt in einer Länge von mindestens 0,5 Millimetern und einem Durchmesser aufweist, der kleiner oder gleich dem Gewindekerndurchmesser ist.

Vorzugsweise beträgt der Gewindedurchmesser 4 bis 8 Millimeter, während die Schraubenlänge vorzugsweise das drei- bis fünffache des Gewindedurchmessers beträgt.

Zwei oder vorzugsweise drei gewindefreie Abschnitte sind vorgesehen. Die Länge jedes gewindefreien Abschnittes beträgt vorzugsweise 1 bis 10 Millimeter.

Ein einzelner gewindefreier Abschnitt ist unmittelbar an einem Schraubenkopf anschließend und am Schraubenende angeordnet.

Der Schraubenkopf weist einen Kopfdurchmesser auf, der gleich oder größer als der Gewindedurchmesser ist. Die Kopfhöhe des Schraubenkopfes weist das 0,1 bis 0,5-fache des Gewindedurchmessers auf. Weiterhin weist der Schraubenkörper eine für einen Eingriff eines Schraubendreherinstruments geeignete Form auf, insbesondere in Form eines im Schraubenkopf angeordneten Innensechskants.

In einer vorteilhaften Ausführungsform weist der Schraubenkörper eine selbstschneidende und/oder selbstbohrende Schraubenspitze auf.

Weiterhin kann bei einer vorteilhaften Ausführungsform eine axiale Kanülierung innerhalb des Schraubenkörpers angeordnet sein. Diese kann beispielsweise zum Einführen eines Drahtes oder einer Injektionsnadel verwendet werden.

Zweckmäßigerweise ist die Knochenschraube aus einem bioverträglichen Material, insbesondere Titan und/oder Titanlegierungen oder bioresorbierbarem Material ausgebildet.

Die erfindungsgemäße Knochenschraube soll nachfolgend anhand beispielhafter Ausführungsformen näher erläutert werden. Zur Verdeutlichung dienen die Figuren 1 bis 3. Es werden für gleiche bzw. gleich wirkende Teile die selben Bezugszeichen verwendet.

Es zeigt:
- Fig. 1: eine erste, nicht erfindungsgemäße, Ausführungsform einer Knochenschraube mit an einem Schraubenkopf anschließenden gewindefreiem Ab- schnitt,
- Fig. 2: eine zweite, nicht erfindungsgemäße, Ausführungsform mit einem weiteren, inmitten des Gewindes angeordneten gewindefreien Abschnitt,
- Fig. 3: eine dritte, nicht erfindungsgemäße Ausführungsform mit einer gewindefreien Schraubenspitze und
- Fig. 4: eine erfindungsgemäße Ausführungsform mit Konuswinkel-Führungsspitze FS

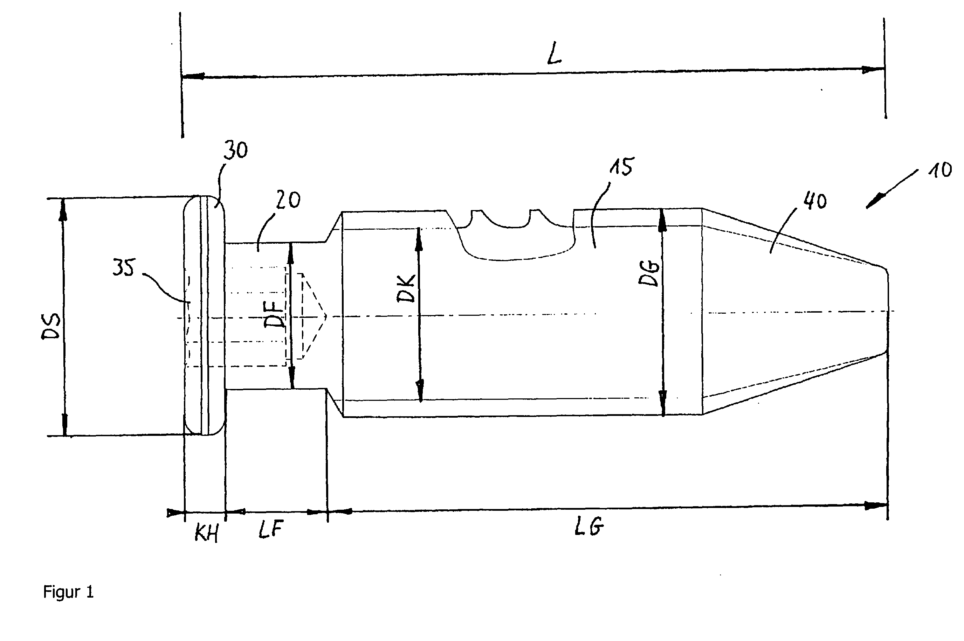

Fig. 1 zeigt eine erste beispielhafte Ausführungsform. Der Schraubenkörper 10 setzt sich zusammen aus einem Gewinde 15, einem gewindefreien Abschnitt 20, einem Schraubenkopf 30 mit einem Innensechskant 35 und einer Schraubenspitze 40, die als selbstschneidende bzw. selbstbohrende Spitze ausgeführt ist. Die Länge L des Schraubenkörpers setzt sich aus der Kopfhöhe KH, der Länge des gewindefreien Abschnitts LF und der Gewindelänge LG zusammen. Das Gewinde 15 weist einen Gewindedurchmesser DG und einen Gewindekerndurchmesser DK auf. Der Durchmesser DF des gewindefreien Abschnitts 20 ist bei der gezeigten Ausführungsform kleiner als der Gewindekerndurchmesser DK. Der Durchmesser DS des Schraubenkopfes ist größer als der Gewindedurchmesser DG des Gewindes.

Bei der in Fig. 1 dargestellten maßstäblich vergrößerten Knochenschraube beträgt die Kopfhöhe KH etwa das 0,25-fache des Gewindedurchmessers DG. Die Schraubenlänge L verhält sich zum Gewindedurchmesser im wesentlichen wie 3,4 : 1. Der gewindefreie Abschnitt 20 schließt in dem dargestellten Beispiel unmittelbar an den Schraubenkopf 30 an.

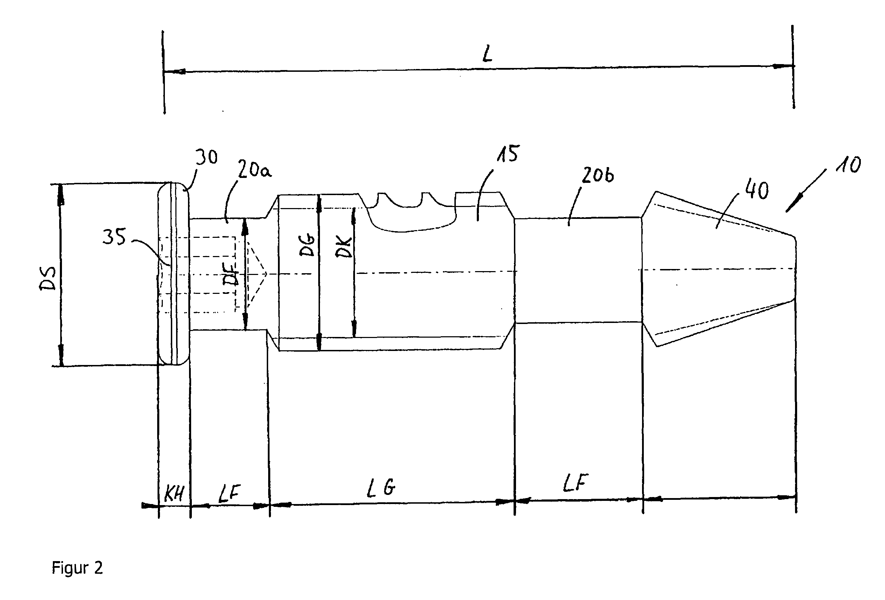

Fig. 2 zeigt eine zweite beispielhafte Ausführungsform. Der Schraubenkörper 10 besteht wiederum aus dem Gewinde 15, einem gewindefreien Abschnitt 20, der in diesem Fall aus zwei Unterabschnitten 20a und 20b besteht, dem Schraubenkopf 30 mit Innensechskant 35 und der selbstschneidend bzw. selbstbohrend ausgebildeten Schraubenspitze 40. Wie bei der vorhergehenden Ausführungsform ist der Durchmesser DF der gewindefreien Abschnitte 20a und 20b kleiner als der Gewindekerndurchmesser DK des Gewindes 15. Aus der Figur ist zu entnehmen, dass die beiden gewindefreien Abschnitte unterschiedliche Durchmesser aufweisen. Der Durchmesser des ersten Unterabschnitts 20a ist bei diesem Ausführungsbeispiel größer als der Durchschnitt des Unterabschnitts 20b. Der Durchmesser DS des Schraubenkopfes ist größer als der Gewindedurchmesser DG des Gewindes.

Bei der in Fig. 2 dargestellten Ausführungsform beträgt die Kopfhöhe KH etwa das 0,2-fache des Gewindedurchmessers DG. Die Schraubenlänge L verhält sich bei dieser Ausführungsform zum Gewindedurchmesser im wesentlichen wie 4,1 : 1.

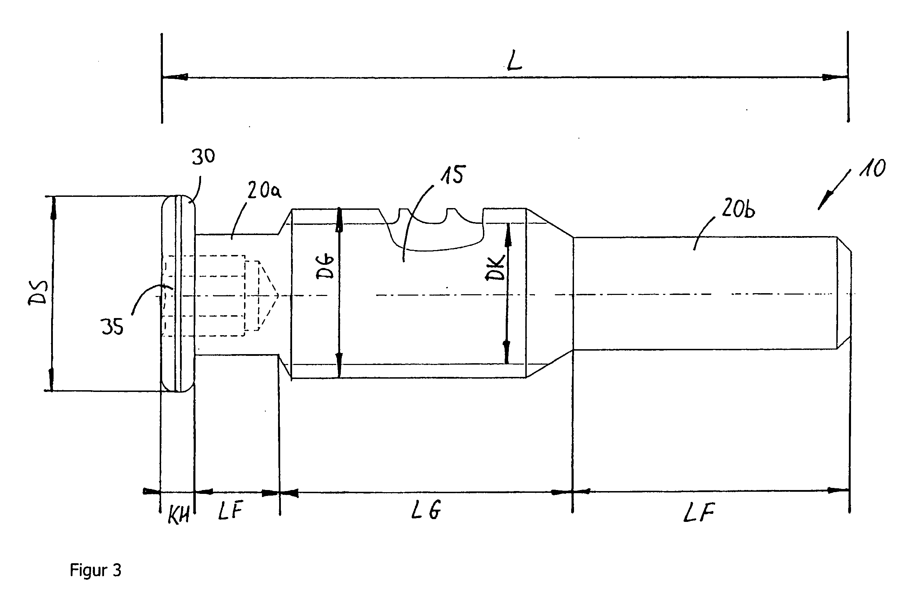

Fig. 3 zeigt eine dritte beispielhafte Ausführungsform. Der Schraubenkörper 10 weist wiederum das Gewinde 15 auf, wobei die gewindefreien Abschnitte 20a und 20b unmittelbar an den Schraubenkopf 30 anschließen bzw. das Schraubenende bilden. Der Schraubenkopf weist wie bei den vorhergehenden Ausführungsformen einen Innensechskant 35 auf. Die Schraubenspitze wird in diesem Fall von einem gewindefreien Abschnitt gebildet und ist demzufolge nicht schneidend oder bohrend. Eine Ausführungsform nach Fig. 3 ist zum Einschrauben in ein bereits vorgebohrtes Loch mit einem Durchmesser vorgesehen, der im wesentlichen dem Gewindekerndurchmesser der Knochenschraube entspricht. Der Durchmesser DF der gewindefreien Abschnitte 20a und 20b sind wie aus der Figur zu entnehmen ist, kleiner als der Gewindekerndurchmesser DK des Gewindes 15. Die beiden gewindefreien Abschnitte können unterschiedliche Durchmesser aufweisen.

Bei der in Fig. 3 gezeigten Ausführungsform beträgt die Kopfhöhe KH des Schraubenkopfes 30 das etwa 0,2-fache des Gewindedurchmessers DG. Bei dem Ausführungsbeispiel aus Fig. 3 beträgt das Verhältnis der Schraubenlänge L zum Gewindedurchmesser DG etwa 4,1 : 1.

Es ist ohne weiteres möglich, die gezeigten Ausführungsformen kanüliert auszubilden. In diesem Fall ist entlang der Längsachse ein kanülenartiger Hohlraum durch den Schraubenkörper geführt. Dabei ist der Innensechskant im Schraubenkopf als Ausformung des kanülenartigen Hohlraums ausgebildet.

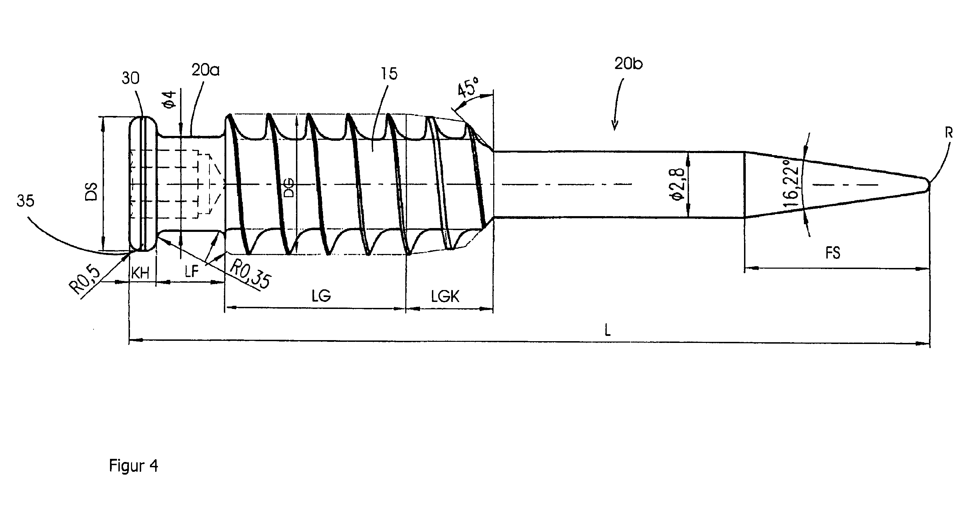

Bei der in Fig. 4 gezeigten, erfindungsgemäßen Ausführungsform ist der Durchmesser im kopffernen Gewindeabschnitt 20b wesentlich kleiner, bevorzugt halb so groß wie der Gewindedurchmesser DG.

Am gewindefreien Abschnitt 20b schließt sich bei dieser Ausführungsform eine Führungsspitze FS mit einem Konuswinkel im Bereich von 14-18°, bevorzugt ca. 16° an.

Diese Führungsspitze FS weist am Konusanfang einen Rundungsradius R auf.

Ergänzend besitzt der Gewindeabschnitt, der sich aus den Bereichen LG und LGK zusammensetzt, zum kopffernen gewindefreien Abschnitt gerichtet einen konischen Verlauf (Abschnitt LGK).

### Bezugszeichenliste

- 10: Schraubenkörper
- 15: Gewinde
- 20: gewindefreier Abschnitt
- 30: Schraubenkopf
- 35: Innensechskant
- 40: Schraubenspitze
- DG: Gewindedurchmesser
- DK: Gewindekerndurchmesser
- DF: Durchmesser des gewindefreien Abschnitts
- DS: Durchmesser des Schraubenkopfes
- KH: Kopfhöhe
- LF: Länge gewindefreier Abschnitt
- LG: Gewindelänge/Gewindeabschnitt
- L: Gesamtlänge
- LGK: konischer Gewindeabschnitt
- FS: Führungsspitze
- R: Rundungsradius

## Patentansprüche

1. Knochenschraube, nämlich Ankerschraube für spongiöses Knochengewebe zur Verankerung von Nahtfäden und für eine Tenodese mit einem Schraubenkörper (10) mit einem Gewinde (15), das einen Gewindedurchmesser (DG) im Bereich von 3mm bis 15mm aufweist und einer dem 2- bis 10-fachen des Gewindedurchmessers entsprechenden Länge (L) des Schraubenkörpers in Verbindung mit einem auf der Längsachse des Schraubenkörpers angeordneten gewindefreien Abschnitt (20a) mit einer Länge (LF) von mindestens 0,5mm, welcher sich unmittelbar an einen Schraubenkopf (30) anschließt, sowie einem weiteren, schraubenkopffernen gewindefreien Abschnitt (20b),
**dadurch gekennzeichnet, dass**
der Durchmesser (DF) des kopfnahen gewindefreien Abschnitts (20a) kleiner oder gleich dem Gewindekerndurchmesser (DK) ist, weiterhin der Durchmesser im kopffernen gewindefreien Abschnitt (20b) circa halb so groß wie der Gewindedurchmesser (DG) ist und sich am kopffernen gewindefreien Abschnitt (20b) eine Führungsspitze (FS) mit einem Konuswinkel im Bereich von 14 - 18° anschließt.

2. Knochenschraube nach Anspruch 1,
**dadurch gekennzeichnet, dass**
die Länge (LF) jedes gewindefreien Abschnitts (20a, 20b) 1mm bis 10mm beträgt.

3. Knochenschraube nach einem der vorangegangenen Ansprüche,
**dadurch gekennzeichnet, dass**
der Schraubenkopf (30) einen Kopfdurchmesser (DS) aufweist, der gleich dem Gewindedurchmesser (DG) ist.

4. Knochenschraube nach einem der vorangegangenen Ansprüche,
**dadurch gekennzeichnet, dass**
der Schraubenkopf (30) eine Kopfhöhe (KH) von einem 0,1- bis 0,5-fachen des Gewindedurchmessers (DG) aufweist.

5. Knochenschraube nach einem der vorangegangenen Ansprüche,
**gekennzeichnet durch**
eine axial innerhalb des Schraubenkörpers (10) verlaufende Kanülierung.

6. Knochenschraube nach einem der vorangegangenen Ansprüche,
**dadurch gekennzeichnet, dass**
die Führungsspitze (FS) am Konusanfang einen Rundungsradius (R) aufweist.

## Claims

1. Bone screw, namely an anchor screw for spongy bone tissue for anchoring sutures and for a tenodesis, comprising a screw body (10) having a thread (15), which has a thread diameter (DG) ranging from 3 mm to 15 mm, and a length (L) of the screw body corresponding to two to ten times the thread diameter in connection with a non-threaded section (20a) located on the longitudinal axis of the screw body and having a length (LF) of at least 0.5 mm, which directly joins a screw head (30), and another non-threaded section (20b) away from the screw head,
**characterized in that**
the diameter (DF) of the non-threaded section (20a) near the head is smaller than or equal to the core diameter of the thread (DK), that further the diameter in the non-threaded section (20b) away from the head has approximately half the size of the thread diameter (DG) and that a guide point (FS) having a cone angle in the range of 14° to 18° joins the non-threaded section (20b) away from the head.

2. Bone screw according to claim 1,
**characterized in that**
the length (LF) of each non-threaded section (20a, 20b) amounts to 1 mm to 10 mm.

3. Bone screw according to one of the preceding claims,
**characterized in that**
the screw head (30) has a head diameter (DS) which is equal to the thread diameter (DG).

4. Bone screw according to one of the preceding claims,
**characterized in that**
the screw head (30) has a height (KH) of 0.1 to 0.5 times the thread diameter (DG).

5. Bone screw according to one of the preceding claims,
**characterized by**
a cannulization extending axially within the screw body (10).

6. Bone screw according to one of the preceding claims,
**characterized in that**
the guide point (FS) has a round radius (R) at the front end of the cone.

## Revendications

1. Vis à os, en particulier vis d'ancrage pour tissu osseux spongieux destinée à l'ancrage de fils de suture et pour une ténodèse, comprenant un corps de vis (10) avec un filetage (15) qui présente un diamètre de filetage (DG) dans la plage de 3 mm à 15 mm et une longueur (L) du corps de vis qui correspond à 2 à 10 fois le diamètre du filetage, en association avec un tronçon (20a) sans filetage, agencé sur l'axe longitudinal du corps de vis avec une longueur (LF) d'au moins 0,5 mm, qui se raccorde directement à une tête de vis (30), ainsi qu'un autre tronçon (20b) dépourvu de pas de vis et éloigné de la tête de vis, **caractérisée en ce que**
le diamètre (DF) du tronçon (20a) dépourvu de pas de vis et proche de la tête, est inférieur ou égal au diamètre en fond de pas de vis (DK), le diamètre du tronçon dépourvu de pas de vis (20b) et éloigné de la tête est environ moitié plus petit que le diamètre (DG) du pas de vis et un tronçon dépourvu de pas de vis (20b) et éloigné de la tête se raccorde à une pointe de guidage (FS) avec un angle conique dans la plage de 14 à 18°.

2. Vis à os selon la revendication 1,
**caractérisée en ce que** la longueur (LF) de chaque tronçon dépourvu de pas de vis (20a, 20b) est entre 1 mm et 10 mm.

3. Vis à os selon l'une des revendications précédentes,
**caractérisée en ce que** la tête de vis (30) présente un diamètre de tête (DS) qui est égal au diamètre du pas de vis (DG).

4. Vis à os selon l'une des revendications précédentes,
**caractérisée en ce que** la tête de vis (30) présente une hauteur de tête (KH) avec un diamètre qui est de 0,1 à 0,5 fois le diamètre du pas de vis (DG).

5. Vis à os selon l'une des revendications précédentes, **caractérisée par** la pose d'une canule qui s'étend axialement à l'intérieur du corps de vis (10).

6. Vis à os selon l'une des revendications précédentes,
**caractérisée en ce que** la pointe de guidage (FS) présente un rayon d'arrondi (R) au départ du cône.
